(19) Europäisches Patentamt
European Patent Office
Office européen des brevets

(11) **EP 1 358 145 B1**

(12) **FASCICULE DE BREVET EUROPEEN**

(45) Date de publication et mention
de la délivrance du brevet:
**02.11.2005 Bulletin 2005/44**

(51) Int Cl.[7]: **C07C 51/10**, C07C 51/12,
C07C 53/08, C07C 67/36,
C07C 67/37, C07C 69/14

(21) Numéro de dépôt: **02703653.2**

(22) Date de dépôt: **05.02.2002**

(86) Numéro de dépôt international:
**PCT/FR2002/000432**

(87) Numéro de publication internationale:
**WO 2002/062739 (15.08.2002 Gazette 2002/33)**

(54) **MODIFICATION DU SYSTEME CATALYTIQUE DANS UN PROCEDE INDUSTRIEL DE FABRICATION D'ACIDE ACETIQUE ET/OU D'ACETATE DE METHYLE**

MODIFIZIERTES KATALYSATORSYSTEM IN EINEM INDUSTRIELLEN PROZESS ZUR HERSTELLUNG VON ESSIGSÄURE UND/ODER METHYLACETAT

MODIFICATION OF THE CATALYTIC SYSTEM IN AN INDUSTRIAL PROCESS FOR MAKING ACETIC AND/OR METHYL ACETATE ACID

(84) Etats contractants désignés:
**AT BE CH CY DE DK ES FI FR GB GR IE IT LI LU MC NL PT SE TR**

(30) Priorité: **08.02.2001 FR 0101690**

(43) Date de publication de la demande:
**05.11.2003 Bulletin 2003/45**

(73) Titulaire: **ACETEX CHIMIE**
**92205 Neuilly sur Seine Cedex (FR)**

(72) Inventeurs:
• **THIEBAUT, Daniel, Marcel**
**F-64230 Lescar (FR)**

• **MARCHAND, Daniel, Henri**
**F-64110 Jurancon (FR)**
• **KALCK, Philippe, Joseph**
**F-31320 Auzeville-Tolosane (FR)**
• **LE BERRE, Carole, Marie**
**F-31120 La Croix Falgarde (FR)**
• **SERP, Philippe, Gilles**
**F-31810 Clermont le Fort (FR)**

(74) Mandataire: **Giraud, Françoise et al**
**Cabinet Beau de Loménie**
**158, rue de l'Université**
**75340 Paris Cedex 07 (FR)**

(56) Documents cités:
**EP-A- 0 618 183          GB-A- 2 315 069**

**Description**

La présente invention concerne le domaine de la fabrication industrielle de l'acide acétique et/ou de l'acétate de méthyle.

Elle a plus particulièrement trait à un procédé de fabrication en continu d'acide acétique et/ou d'acétate de méthyle au cours duquel on modifie de façon particulièrement douce et régulière, la composition du système catalytique, ce qui permet de passer industriellement d'un système catalytique à base de rhodium seul à un système catalytique à base de rhodium et d'iridium, voire même à base d'iridium seul, et cela, sans avoir à arrêter l'unité de production pour procéder à ce changement de catalyseur.

On connaît de nombreux procédés industriels de fabrication d'acide acétique et/ou d'acétate de méthyle en phase liquide sous pression mettant en oeuvre un système catalytique homogène.

L'invention s'applique tout particulièrement à la modification des procédés mettant en oeuvre initialement la carbonylation du méthanol ou d'un dérivé carbonylable du méthanol en présence d'un catalyseur à base de rhodium.

Les brevets de Paulik et al., qui constituent la base de la technologie ou procédé dit "Monsanto", décrivent la carbonylation de réactifs, en phases liquide ou vapeur, par catalyses homogène ou hétérogène, respectivement par le rhodium (US 3 769 329) ou l'iridium (US 3 772 380). Dans cette technologie, les réactifs sont constitués d'alcools ROH, d'éthers R'-O-R', d'esters R'C(O)OR' et d'halogénures R-X dont le nombre total de carbone est inférieur ou égal à 20. La réaction de carbonylation est conduite en présence d'un de ces réactifs, de monoxyde de carbone (pression partielle de CO entre 1 et 15.000 psig), d'un système catalytique comprenant le rhodium ou l'iridium en présence d'un promoteur halogéné et d'eau, à une température comprise entre 50 et 300°C.

Les premiers procédés de carbonylation développés étaient catalysés par un composé du rhodium, en présence d'un halogénure de méthyle comme co-catalyseur, de préférence l'iodure de méthyle. Ces procédés ont été régulièrement améliorés pour augmenter les vitesses de réaction (donc la productivité du procédé), diminuer la production des impuretés, augmenter la stabilité du catalyseur en solution ainsi que pour réduire les coûts de fabrication.

En particulier, la réduction de la teneur en eau a pu être réalisée par l'ajout de quantités importantes d'ions iodure dans le milieu réactionnel (supérieur à 0,3 mole/litre dans le brevet FR 2 551 434) et/ou par maintien de concentrations imposées des constituants de ce même milieu réactionnel (EP 0 161 874 et EP 0 250 189).

Récemment, les études menées pour remplacer le rhodium par l'iridium, ont abouti à de nouveaux procédés dits "basses teneurs en eau", catalysés à l'iridium seul (EP 0 618 184, EP 0 616 997, EP 0 786 447) ou à l'iridium accompagné de copromoteurs, principalement ruthénium, osmium ou rhénium (EP 0 643 034, EP 0 728 729, EP 0 752 406).

Classiquement ces différents procédés de carbonylation en phase liquide et catalyse homogène sont mis en oeuvre dans une installation comprenant 3 zones distinctes. Dans la 1ère zone, dite zone de réaction, on effectue, sous pression, par introduction simultanée des 2 réactifs, la carbonylation du méthanol ou d'un dérivé carbonylable du méthanol par le monoxyde de carbone. Le milieu réactionnel est maintenu dans une composition donnée par introduction des deux réactifs précédents et recyclages de flux provenant des zones n° 2 et 3 en aval. Dans la zone n° 2, dite zone de vaporisation ou zone de flash, le milieu réactionnel est partiellement vaporisé à une pression inférieure à celle de la zone de réaction n° 1 ; la détente est réalisée avec ou sans apport de chaleur. La fraction vaporisée constituée principalement des réactifs non réagis, d'eau, d'iodure de méthyle, d'acétate de méthyle et d'acide acétique est transférée vers la zone n° 3 dite zone de séparation par distillation et de purification de l'acide acétique et/ou de l'acétate de méthyle produits. La fraction non vaporisée issue de la zone n° 2 de flash composée essentiellement d'acide acétique et du catalyseur est recyclée vers la zone n° 1 de réaction. La zone n° 3, classiquement constituée de 3 colonnes à distiller permet la séparation des différents composants, le recyclage de ceux nécessaires à la conduite de la réaction et la production d'acide acétique et/ou d'acétate de méthyle purifiés. En outre, ces procédés comprennent une section de traitement des gaz et/ou évents. Pour plus de précisions, on pourra se reporter à l'article de M. J. Howard et al, CATALYSIS TODAY, 18 (1993) 325-354.

Plus récemment encore, des procédés de carbonylation employant des systèmes catalytiques, composés à la fois de rhodium et d'iridium, ont été décrits.

Le brevet EP 0 618 183 sur la carbonylation d'alcools ou de dérivés montre la synergie d'un système catalytique composé à la fois de rhodium et d'iridium par rapport à ceux constitués d'un seul métal, rhodium ou iridium. Ce procédé est mis en oeuvre en présence de faibles concentrations de stabilisant des catalyseurs type sels d'iodure solubles (0 à 5 %, de préférence 0 à 2 %) voire en l'absence de tels composés.

Les sels d'iodure solubles sont constitués des iodures de métaux alcalins, alcalino-terreux, d'iodures d'ammonium ou de phosphonium quaternaires.

Le brevet FR 2 750 984 décrit l'amélioration de la consommation du monoxyde de carbone par l'introduction d'un 2ème réacteur de carbonylation situé entre la 1ère zone (réacteur principal) et la 2ème zone de vaporisation (flash) ; le système catalytique est composé d'iridium éventuellement en combinaison avec du rhodium.

Le brevet FR 2 750 985 porte sur la carbonylation du méthanol et propose un procédé de stabilisation du système catalytique composé de complexes solubles d'iridium et de rhodium consistant à maintenir une concentration en eau supérieure à 0,5 % dans la fraction liquide non vaporisée de la 2$^{ème}$ zone de vaporisation (flash) recyclée vers la 1$^{ère}$ zone de réaction.

La demande internationale de brevet WO 00/27785 montre la synergie d'un système catalytique composé d'iridium et de platine et éventuellement de rhodium par rapport à ceux constitués d'un seul métal. Cette demande préconise de maintenir un rapport molaire entre les sels d'iodure solubles introduits et l'iridium inférieur à 10.

La demande internationale de brevet WO 00/78700 concerne la production d'acide acétique par réactions simultanées d'isomérisation du formiate de méthyle et de carbonylation du méthanol en présence d'iridium et éventuellement de rhodium. Cette demande enseigne la stabilisation du système catalytique par le maintien de la concentration en radicaux formyles (formiate de méthyle + acide formique) supérieure à 1 % dans la fraction non vaporisée de la 2$^{ème}$ zone de vaporisation (flash). Il est recommandé de maintenir des iodures sous forme ionique soluble dans ce milieu, sans en préciser le domaine des concentrations.

La demande internationale de brevet WO 00/24701 décrit la carbonylation du méthanol avec un système catalytique composé à la fois de rhodium et d'iridium, en présence d'ions iodure comme stabilisants/copromoteurs de ces catalyseurs. La composition du milieu réactionnel est maintenue constante : eau < 14 % (de préférence 0,1 à 8 %) ; iodure de méthyle 5 à 30 % ; acétate de méthyle 0,5 à 30 % ; iodures 2 à 20 % (de préférence iodure de lithium) ; catalyseurs 100 à 5000 ppm pour chaque métal ; le système catalytique peut, en outre, comprendre un sel d'un métal de transition, de préférence le ruthénium.

Les brevets et demandes de brevets ci-dessus décrivent des milieux réactionnels de carbonylation établis à base d'un système catalytique composé à la fois de rhodium et d'iridium et dont la composition a été optimisée pour atteindre les résultats attendus de ces inventions. Aucun de ces documents n'indique ou ne suggère la manière de passer d'un système catalytique composé de rhodium seul à un système catalytique basé sur le mélange des catalyseurs rhodium + iridium.

Par ailleurs, dans un tel changement, il est bien connu qu'il faut se préoccuper du rôle joué par les métaux de corrosion, couramment et classiquement présents dans les milieux réactionnels de carbonylation (fer, nickel, chrome, molybdène, tungstène, zirconium et tout métal provenant de la corrosion des équipements industriels et qui peuvent être recyclés vers le réacteur en cours de production). Ils sont, comme cela apparaît en particulier dans le brevet FR 2 551 434, en général globalement jugés néfastes en catalyse au rhodium seul, car ils accélèrent la réaction de conversion ou de gaz à l'eau (encore désignée sous l'abréviation WGSR pour Water Gas Shift Reaction) :

$$H_2O + CO \rightarrow H_2 + CO_2$$

Toujours en catalyse au rhodium seul, le brevet EP 0 384 652 plus sélectif, préconise d'enlever le fer et le nickel pour ne laisser que les métaux du groupe VIb (Mo, W, Cr) ayant une influence positive sur la vitesse de carbonylation et donc la productivité du procédé. Une variante consiste à ajouter ces métaux de corrosion du groupe VIb dans le milieu réactionnel de carbonylation.

Par contre, en catalyse à l'iridium seul, le problème posé par les métaux de corrosion semble plus simple : il faut limiter leur concentration dans le milieu réactionnel à quelques centaines de ppm (brevet FR 2 750 984) voire à une valeur inférieure à 200 ppm (demandes de brevet WO 00/27785 et WO 00/78700) pour limiter :

- D'une part, la réaction de gaz à l'eau, comme pour le rhodium,
- D'autre part, la concentration en ions iodure, considérés comme des poisons dans la carbonylation catalysée par l'iridium. En effet, ces ions iodure, provenant de différentes origines, sont en partie amenés par les sels que représentent les iodures des métaux de corrosion. Le brevet FR 2 750 984 et la demande WO 00/27785 proposent de maintenir inférieur à 10 le rapport molaire entre les ions iodure et l'iridium.

Comme on le constate de ce qui précède, au vu, à la fois de l'influence différente, selon le système catalytique, de la concentration en métaux de corrosion ainsi que de la concentration en ions iodure, dans le cas des procédés de fabrication en continu d'acide acétique par carbonylation du méthanol ou d'un de ses dérivés carbonylables, la seule solution qu'envisage l'homme du métier à ce jour, s'il veut changer de catalyseur pour passer d'un système catalytique à base de rhodium à un système catalytique à base de rhodium nettement enrichi en iridium ou d'iridium seul est d'arrêter l'installation pour modifier le milieu réactionnel, de façon en particulier à régler la concentration en métaux de corrosion et en iodures à des valeurs qui ne perturbent pas la bonne marche du procédé avec le nouveau système catalytique.

Or, contrairement à toute attente, les inventeurs de la présente invention ont maintenant montré qu'il était possible de modifier sans arrêt de l'installation, la composition du catalyseur en ajoutant un catalyseur à base d'iridium, de manière régulière et croissante à un milieu réactionnel de carbonylation catalysée à l'origine par un catalyseur à base de rhodium et possédant la composition classique et connue selon la technologie Monsanto, c'est à dire un milieu réactionnel contenant au moins 14 % d'eau, au plus 15 % d'iodure de méthyle, au plus 2 % d'acétate de méthyle et quelques centaines de ppm de rhodium, en présence de quelques milliers de ppm de métaux de corrosion, le reste étant constitué par de l'acide acétique.

Ainsi donc, la présente invention propose de modifier le système catalytique dans un procédé industriel de fabrication d'acide acétique conventionnel dit procédé de type Monsanto catalysé au rhodium par remplacement au moins partiel du rhodium par de l'iridium, et cela :

- sans arrêter le procédé pour effectuer le changement du système catalytique rhodium seul pour le système rhodium + iridium,
- sans modifier profondément la composition du milieu réactionnel en particulier les ions iodures naturellement présents et les métaux de corrosion,
- sans altérer la stabilité du système catalytique,
- en maintenant, voire en améliorant la vitesse de carbonylation donc la productivité du procédé,
- en réduisant les coûts de fabrication par diminution de la teneur en eau du milieu réactionnel entraînant une économie d'énergie aux distillations.

Mais d'autres avantages apparaîtront plus clairement à la lecture de la description détaillée qui va suivre.

Il est en particulier apparu que la modification de système catalytique pouvait se faire de façon particulièrement douce et sans arrêt de l'installation, ce qui représente un premier avantage économique puisqu'il permet d'éviter l'arrêt complet de l'installation pour modifier le milieu réactionnel. Il est apparu en plus qu'il était possible, au cours de cette modification du système catalytique, pour l'enrichir en iridium, d'abaisser considérablement la teneur en eau, ce qui constitue un deuxième avantage économique considérable du procédé de l'invention. Par ailleurs, cette modification de la teneur en eau du milieu réactionnel permet d'envisager, dans des conditions industrielles, de réaliser simultanément, avec la carbonylation initiale, une réaction d'isomérisation du formiate de méthyle conduisant, elle aussi, à la formation d'acide acétique et/ou d'acétate de méthyle, voire même de limiter à terme la production d'acide acétique et/ou d'acétate de méthyle à la seule production par réaction d'isomérisation du formiate de méthyle, toutes ces modifications se faisant sans arrêt de l'installation, ce qui présente un avantage économique considérable.

Ainsi, selon l'une de ses caractéristiques essentielles, l'invention concerne un procédé de fabrication en continu d'acide acétique et/ou d'acétate de méthyle, caractérisé en ce que, à partir d'un procédé industriel continu, dit procédé initial, de carbonylation du méthanol ou d'un dérivé carbonylable du méthanol, tel que le diméthyléther, les halogénures de méthyle ou l'acétate de méthyle, en phase liquide homogène et sous pression de monoxyde de carbone, en présence d'un système catalytique comprenant un catalyseur homogène à base de rhodium et un promoteur halogéné, et en présence d'une concentration en eau dans le milieu réactionnel supérieure ou égale à 14 %, on réalise pendant le fonctionnement continu de l'installation, une modification progressive de la composition dudit catalyseur homogène par additions au cours du temps, d'un composé d'iridium.

Le procédé de l'invention consiste donc à modifier progressivement, au cours du temps, la composition du système catalytique en partant d'un procédé de fabrication, dit procédé initial, qui est un procédé dérivé du procédé Monsanto, c'est à dire un procédé selon lequel l'acide acétique et/ou l'acétate de méthyle sont fabriqués en continu par carbonylation de méthanol ou d'un dérivé carbonylable du méthanol en phase liquide sous pression, en présence d'un catalyseur homogène contenant du rhodium.

Le procédé de l'invention comprend donc une étape essentielle, ci-après désignée par phase de modification, au cours de laquelle on modifie le système catalytique en ajoutant un composé à base d'iridium sans arrêter la production, c'est à dire sans arrêter l'installation, sans avoir à la vidanger, sans avoir à la laver ou à la nettoyer ou à la conditionner de façon quelconque pour effectuer le changement de système catalytique.

Le procédé de l'invention, comme exposé précédemment, s'applique à tout procédé initial dérivé du procédé Monsanto fonctionnant initialement avec un système catalytique comprenant un catalyseur homogène à base de rhodium et un promoteur halogéné qui peut être en particulier l'iodure de méthyle.

Ce système de départ, comme tout système de type Monsanto fonctionne avec un milieu réactionnel contenant une teneur en eau supérieure ou égale à 14 %.

La phase de modification du procédé selon l'invention peut s'échelonner sur des durées qui pourront être variables en fonction de la composition du système catalytique visé et s'échelonnera en moyenne sur une durée de quelques mois à environ 5 ans.

La fin de la phase de modification du procédé de l'invention permet de passer d'un système catalysé par du rhodium seul à un système catalysé par un catalyseur homogène à base d'un mélange de rhodium et d'iridium, voire un système catalysé par de l'iridium seul.

La fin de la phase de modification du procédé sera aisément déterminée par l'homme du métier à la fois en fonction d'essais d'orientation du type de ceux réalisés dans la section "exemples" du présent document mais également en fonction des résultats obtenus au cours de la phase de modification et, avec pour optique, d'améliorer à la fois la productivité et la cinétique du procédé de fabrication de l'acide acétique et/ou de l'acétate de méthyle.

Ainsi, après cette phase de modification, on peut poursuivre ensuite la fabrication en continu de l'acide acétique et/ou de l'acétate de méthyle en maintenant la composition du système catalytique tel que modifié dans la phase de modification. Cette nouvelle phase sera ci-après désignée par phase de stabilisation.

Dans cette phase, suivant la phase de modification, on maintient les concentrations en rhodium et en iridium dans le milieu réactionnel par ajout de composés de rhodium et/ou de composés d'iridium, et cela, pour compenser, comme cela est classique dans le métier, les pertes en catalyseur au cours des opérations de fabrication en continu, en particulier des pertes dues à des entraînements éventuels du catalyseur difficilement évitables malgré les différents recyclages effectués dans un procédé industriel.

Il est courant et classique, dans un procédé de carbonylation catalysé par le rhodium seul (ou l'iridium seul) de maintenir ou d'ajuster l'activité catalytique dans la zone réactionnelle, par des ajouts plus ou moins fréquents de rhodium (ou d'iridium). Ces ajouts sont, par exemple, destinés à maintenir constante, la concentration du catalyseur actif dans le milieu réactionnel pour compenser les pertes en catalyseur par insolubilisation (catalyseur inactif) ou par entraînement vésiculaire dans les vaporisations et distillations classiquement réalisées dans des zones de flash et de distillation/purification.

Dans le cas de la présente invention, les différentes additions de composés d'iridium, au cours de la phase de modification, peuvent être réalisées de différentes façons, elles peuvent être réalisées en particulier de façon régulière ou non, continues ou discontinues, mais de toute façon, sans arrêter la production.

Selon une variante de l'invention, les additions sont réalisées sous forme d'iridium préalablement solubilisé, en particulier sous forme d'iridium solubilisé dans toute solution compatible avec le milieu réactionnel. Cette variante est particulièrement avantageuse car très facile à mettre en oeuvre industriellement. La solution peut être réalisée à partir de composés solides classiquement utilisés dans les procédés catalysés par l'iridium (métal, oxydes ou hydroxydes, sels complexes etc). Cette solubilisation peut avoir lieu par tous les moyens connus de l'homme du métier et selon les règles de l'art, en particulier en utilisant des procédés tels que décrits dans les brevets EP 0657386 et EP 0737103 qui donnent les moyens de préparer des solutions catalytiques directement injectables et utilisables dans les réactions de carbonylation.

On privilégiera, d'une façon générale, les modes opératoires de mise en solution de l'iridium compatibles avec des installations industrielles utilisées pour la mise en solution du rhodium dans les procédés conventionnels de carbonylation.

Selon une autre variante, le composé d'iridium peut être introduit sous forme d'un dérivé solide de l'iridium que l'on solubilise in situ dans le milieu réactionnel.

Dans un tel cas, le dérivé d'iridium est introduit directement dans le réacteur et sa mise en solution ainsi que la synthèse des espèces catalytiquement actives s'effectuent dans le milieu réactionnel, en présence de promoteur halogéné, en particulier d'iodure de méthyle et sous pression de monoxyde de carbone.

On aboutit ainsi dans les tous cas, à une réaction de carbonylation catalysée par un mélange de rhodium et d'iridium, dont les concentrations dans le milieu réactionnel sont ajustables à tout instant par addition de rhodium ou d'iridium ou des deux à la fois.

Ainsi donc, comme exposé précédemment, l'introduction du composé d'iridium dans le milieu réactionnel peut se faire sous différentes formes physiques.

Elle peut également être réalisée selon différentes variantes.

Selon une première variante, les ajouts plus ou moins fréquents de rhodium faits dans le procédé initial sont simplement remplacés par des additions correspondantes d'iridium pendant toute la phase de modification.

Ainsi donc, selon cette variante, les additions de composés d'iridium sont réalisées de façon à compenser les pertes en rhodium en remplaçant le rhodium perdu par de l'iridium.

Selon une autre variante, l'addition du composé d'iridium est réalisée en continu.

Selon une autre variante encore, les additions d'iridium sont réalisées en discontinu.

Dans tous les cas, comme exposé précédemment, les essais d'orientation et le suivi à la fois de la cinétique de réaction et de la productivité du système permettront de déterminer l'arrêt de la phase de modification et le passage à la phase de stabilisation du système catalytique au rapport molaire Ir/Rh déterminé.

Dans les différentes variantes d'introduction du composé d'iridium, une fois les proportions désirées atteintes dans le système catalytique, on pourra maintenir ou ajuster les concentrations du milieu réactionnel en rhodium et iridium, par addition de composés de rhodium et/ou d'iridium.

Selon une variante particulièrement avantageuse de l'invention lorsque il s'avère nécessaire d'ajouter à la fois du rhodium et de l'iridium pour maintenir et/ou ajuster la concentration du milieu réactionnel en rhodium et iridium, on procède à l'introduction simultanée de ces deux métaux catalytiques sous forme d'une solution préalablement préparée les renfermant. Une telle technique présente de nombreux avantages et en particulier celui de simplifier la technique opératoire et de réduire les coûts puisqu'elle permet d'éviter la préparation séparée de deux solutions catalytiques et leur introduction séparée dans le milieu réactionnel, en permettant de réaliser l'ajout simultané de rhodium et d'iridium.

La solution catalytique pourra être préparée par tout moyen permettant de réaliser l'ajout simultané de rhodium et d'iridium sous forme d'une solution catalytique dans laquelle la mise en solution du rhodium et de l'iridium est réalisée conjointement et simultanément à partir des composés de rhodium et des composés d'iridium.

Il ressort des exemples réalisés par les inventeurs de la présente invention, que la mise en solution simultanée du rhodium et de l'iridium, en particulier sous forme d'iodure de rhodium et d'iodure d'iridium, peut être réalisée dans des conditions classiques du type de celles décrites dans le procédé Monsanto pour l'iodure de rhodium seul.

D'une façon générale, aussi bien dans la phase de modification que dans la phase de stabilisation du système catalytique, on maintient avantageusement, dans le milieu réactionnel, une concentration molaire en rhodium comprise entre 0,1 et 50 mmole/l et une concentration molaire en iridium comprise entre 0,1 et 25 mmole/l.

Par ailleurs, le rapport atomique iridium/rhodium est avantageusement maintenu à une valeur comprise entre 1/99 et 99/1.

Il est également possible, comme exposé précédemment, de poursuivre les additions de composé d'iridium dans la phase de modification jusqu'à obtention d'iridium comme seul métal dans le système catalytique homogène.

Le procédé de l'invention s'applique tout particulièrement à des procédés de fabrication en continu d'acide acétique et/ou d'acétate de méthyle réalisés dans une installation comprenant une zone I de réaction où la température est maintenue à une valeur comprise entre 150 et 250°C et la pression totale est comprise entre $5.10^5$ Pa et $200.10^5$Pa, une zone II de flash où le produit issu de la zone I de réaction est partiellement vaporisé à une pression inférieure à celle régnant dans la zone I de réaction, la fraction non vaporisée issue de cette zone II de flash étant recyclée vers la zone I de réaction et la fraction vaporisée étant purifiée dans une zone III de séparation par distillation et purification de l'acide acétique et/ou de l'acétate de méthyle formés.

Les différentes injections de composés d'iridium, quelque soit leur forme physique, peuvent être réalisées de différentes manières comme exposé précédemment mais également à différents endroits d'une installation du type de celles décrites ci-dessus comprenant en particulier, trois zones principales.

Selon une variante, les composés d'iridium, solides ou en solution, peuvent être introduits directement dans le milieu réactionnel de la zone I de réaction ou dans la fraction non vaporisée de la zone II de flash ou dans la canalisation de recyclage de cette fraction non vaporisée de la zone II vers la zone I, cette dernière variante étant préférée.

Ainsi donc, les composés d'iridium peuvent être injectés, soit directement dans le milieu réactionnel de la zone I, soit dans un flux recyclé des zones II ou III vers la zone I de réaction.

Comme exposé précédemment, il est inutile d'éliminer, plus que de coutume, les métaux de corrosion pour pratiquer les additions d'iridium.

Les règles de l'art des procédés conventionnels fixent à une valeur de 5000 mg/kg (ppm), la teneur en métaux de corrosion à ne pas dépasser dans le milieu réactionnel.

Cette règle pourra être conservée pour les additions d'iridium et donc pour la conduite du procédé de carbonylation pendant toute la durée de la phase de modification mais également pendant la phase de stabilisation.

Le maintien de cette concentration en métaux de corrosion à une valeur inférieure ou égale à 5000 mg/kg peut s'effectuer par tous les moyens classiques connus de l'homme de métier et déjà largement appliqués dans les procédés catalysés par le rhodium seul ou l'iridium seul :

- précipitation sélective,
- extraction liquide-liquide,
- passage sur résine échangeuse d'ions,
- osmose,
- traitement sur membrane sélective...

Comme déjà exposé précédemment, outre les intérêts techniques et pratiques, l'invention s'accompagne d'économies et d'améliorations des coûts par rapport à la méthode classique qui consiste à arrêter la fabrication pour vidanger l'installation, éliminer le milieu réactionnel au rhodium et le remplacer par un milieu réactionnel différent et spécifique du couple rhodium + iridium ou de l'iridium seul.

Ceci permet d'éviter les pertes de production pendant l'arrêt de l'installation, les pertes ou difficultés de récupération du rhodium, les difficultés de redémarrage de l'installation liées à ce que la conduite de la nouvelle installation, fonctionnant avec un système catalytique mixte à base d'iridium et de rhodium, est différente de celle de l'ancienne fonctionnant au rhodium seul.

Comme exposé précédemment, un autre avantage particulièrement intéressant de la présente invention est qu'elle permet de réduire la teneur en eau du milieu réactionnel en dessous des valeurs conventionnelles de 14 % par rapport au poids du milieu réactionnel, une fois les additions d'iridium réalisées.

Cette teneur en eau pourra être même abaissée jusqu'à des valeurs inférieures à 5 % sans altérer les performances du procédé de carbonylation catalysé par le mélange rhodium + iridium, ce qui présente un intérêt supplémentaire.

En effet, il est bien connu de l'homme du métier, que la vitesse de carbonylation augmente avec l'élévation de la teneur en eau du milieu réactionnel conventionnel au rhodium seul. La vitesse de carbonylation atteint le maximum pour les valeurs de la concentration en eau de l'ordre de 14 à 15 % (10 moles/l), comme indiqué dans le brevet EP 0055618 et la publication de HJORTKJAER et JENSEN, Ind. Eng. Chem. Prod. Res. Dev, volume 16, n°4,1977,281-285, et reste constante, même pour des teneurs en eau supérieures à 14 ou 15 %. La possibilité, grâce à l'invention, de réduire la teneur en eau dans le cas d'un système catalytique, à base de rhodium et d'iridium, en conservant les mêmes vitesses de carbonylation que celles obtenues avec le rhodium à 14 % d'eau permet de réduire de façon notable, les coûts énergétiques de l'élimination de l'eau dans la zone III de séparation/distillation/purification de l'acide acétique et/ou de l'acétate de méthyle produits.

Un avantage supplémentaire de pouvoir abaisser la teneur en eau, dans le milieu réactionnel et en particulier de pouvoir l'abaisser à une valeur inférieure à 5 % en poids par rapport au poids de ce milieu réactionnel, est que cela permet d'envisager la fabrication complémentaire d'acide acétique et/ou d'acétate de méthyle par réaction simultanée d'isomérisation de formiate de méthyle sous pression de monoxyde de carbone, en plus de la carbonylation du méthanol. Cela permet en particulier d'augmenter la production d'acide acétique dans une installation existante sans disposer de monoxyde de carbone supplémentaire et de réaliser des économies d'énergie importantes lors des étapes de distillation.

Ainsi, dès que la valeur de la concentration en eau dans le milieu réactionnel sera descendue à une valeur inférieure ou égale à 5 % en poids par rapport au poids du milieu réactionnel, on pourra aisément introduire du formiate de méthyle dans le milieu réactionnel et réaliser simultanément les réactions de carbonylation du méthanol ou du dérivé carbonylable et d'isomérisation du formiate de méthyle sous pression de monoxyde de carbone, comme cela ressort de la demande internationale WO 97/35828.

Il sera même possible, dans ces conditions, d'arrêter progressivement l'introduction du méthanol ou du dérivé carbonylable du méthanol dans le milieu réactionnel pour passer à un système fonctionnant par isomérisation seule du formiate de méthyle sous pression de monoxyde de carbone (comme cela ressort de la demande internationale WO 97/35829).

Enfin, il est apparu que, lorsque le système catalytique initialement à base de rhodium a été modifié selon la présente invention, c'est à dire transformé dans des conditions particulièrement douces, en un système catalytique à base d'un mélange de rhodium et d'iridium, voire même en un système catalytique constitué exclusivement d'iridium, il est encore possible de modifier ce système catalytique pour y introduire progressivement un composé de platine, de façon à passer progressivement à un procédé de fabrication d'acide acétique et/ou d'acétate de méthyle en présence d'un catalyseur homogène comprenant des proportions d'iridium et de platine permettant d'obtenir les avantages décrits dans la demande de brevet WO 0027785. Dans un tel cas, la concentration en platine est avantageusement maintenue à une valeur comprise entre 1 mmole et 25 mmole par litre de milieu réactionnel.

## EXEMPLES

On a réalisé des séries d'essais sur des solutions synthétiques (milieu réactionnel de carbonylation reconstitué) et un milieu réactionnel industriel réel de carbonylation (échantillons de 50 ml prélevés directement à partir du réacteur de carbonylation de la zone I de réaction) en tout point identiques entre chaque série à l'exception de la composition du système catalytique et/ou de la concentration en métaux de corrosion et/ou de la concentration en eau.

Sauf indications contraires, les quantités de catalyseur (Rh ou Ir) sont indiquées [Rh] ou [Ir] et exprimées en millimoles (mmole).

Les résultats de ces différentes séries d'essais sont donnés pour chaque essai par la vitesse de carbonylation calculée par rapport à la consommation de CO mesurée après 10 minutes de réaction (Vcarb 10') correspondant à la quantité de radicaux acétyles formés (acide acétique + acétate de méthyle). Vcarb. est exprimée en mole/litre de milieu réactionnel final et par heure = mole/(l.h.).

La stabilité du système catalytique est appréciée par l'observation de la masse réactionnelle finale -après refroidissement à température ambiante et dégazage à pression atmosphérique- par la présence ou l'absence de dépôt métallique ou de noir de métal.

## 1. MODES OPERATOIRES-TYPE

### 1.1 Mode opératoire pour les solutions synthétiques

➤ Préparation de la solution catalytique :

Dans un autoclave de 100 ml en Hastelloy® B2, on introduit l'iodure de rhodium, l'iodure d'iridium, les métaux de corrosion, 9 g d'acide acétique pur et 3 g d'eau. L'autoclave est pressurisé sous 5 bar absolus de CO à température ambiante. On porte la température à 190°C, ce qui nécessite environ 1 heure et on laisse 10 minutes à 190°C sous pression autogène (10 bar).

➤ Réaction de carbonylation (effectuée immédiatement ensuite).

Par un réservoir placé au-dessus de l'autoclave, on injecte sous pression de CO un mélange composé d'eau, d'iodure de méthyle, d'acétate de méthyle, d'acide acétique et de méthanol. La composition du milieu réactionnel synthétique hors méthanol est la suivante : Eau 14 %, iodure de méthyle 9 %, acétate de méthyle 2 %, rhodium, iridium, métaux de corrosion suivant essai, acide acétique qsp 100 %. Le poids de milieu réactionnel synthétique hors méthanol est d'environ 56-57 g et le méthanol ajouté représente 10 % de ce poids soit 5,6 g.

La température est remontée à 190°C et la réaction de carbonylation conduite sous 30 bar absolus par injection de CO, à 190 ± 0,5°C, pendant 10 minutes. L'autoclave est refroidi, vidangé et la présence ou l'absence de dépôt métallique est notée.

➤ Expression des données dans les tableaux

Les concentrations en catalyseurs, Rh et Ir, sont exprimées en millimoles de métal concerné dans le milieu réactionnel de l'essai.

Les métaux de corrosion [MétCor] sont introduits sous forme d'iodure métallique ou de métal carbonyle ; la concentration totale est exprimée en mg/kg (ppm) par rapport au poids de milieu réactionnel sans le méthanol (56-57 g). La répartition pondérale des métaux de corrosion est la suivante :

Fer : 20 % ; nickel : 30 % ; chrome : 20 % ; molybdène : 30 %.

### 1.2 Mode opératoire pour le milieu réactionnel industriel

➤ Préparation du milieu réactionnel de carbonylation

Des échantillons de 50 ml sont prélevés en flacons étanches par la prise analytique d'un réacteur industriel de carbonylation du méthanol en catalyse rhodium seul et conservés à l'abri de la lumière à température ambiante. La prise analytique est située sur la canalisation reliant le réacteur -zone I- au flash -zone II- près du réacteur, le prélèvement est effectué de façon étanche par un système DOPAK®). 50 ml de milieu réactionnel industriel sont introduits dans un autoclave de 100 ml en Hastelloy® B2 (soit environ 56-57 g).

Le milieu réactionnel industriel possède la composition suivante : eau 14 %, iodure de méthyle 9 %, acétate de méthyle 2 %, rhodium 600 mg/kg, métaux de corrosion (naturellement présents dans le milieu réactionnel industriel du fait de la corrosion des matériaux des équipements industriels) 5000 mg/kg (fer 1000, nickel 1500, chrome 1000, molybdène 1500 mg/kg), acide acétique qsp 100 %. On introduit suivant les essais, l'iodure d'iridium.

L'autoclave est fermé et pressurisé par 5 bar absolus de CO à température ambiante.

On chauffe à 190°C sous pression autogène (10 bar), ce qui nécessite environ 1 h et on laisse 10 minutes à 190°C. On ajoute alors par le réservoir sous pression de CO environ 5,6 g de méthanol (soit environ10 % de la masse réactionnelle). On chauffe à 190°C et la réaction de carbonylation est conduite sous 30 bar absolus par injection de CO, à 190 ± 0,5°C, pendant 10 minutes. L'autoclave est refroidi, vidangé et la présence ou l'absence de dépôt métallique est notée.

➤ Expression des données dans les tableaux (voir 1.1 ci-dessus)

## 2. ESSAIS AVEC DES SOLUTIONS SYNTHETIQUES

### 2.1 Concentration en métaux de corrosion nulle

Ces essais sont réalisés en appliquant le mode opératoire type donné en 1.1.

Ces essais réalisés en absence de métaux de corrosion avec ([MétCor] = 0) sont considérés comme des essais comparatifs destinés à servir de référence dans les essais suivants où [MétCor] > 0. Néanmoins, ces essais montrent l'influence d'ajouts croissants d'iridium de 0 à 0,8 millimole pour une concentration en rhodium constante de 0,33-0,34 millimole. La vitesse de carbonylation constante pour les rapports molaires Rh/Ir de 100/0 à 70/30 à 5-6 moles /l.h augmente ensuite à 7,5 et 10 moles/l.h pour les rapports molaires Rh/Ir 50/50 et 30/70.

Les essais en présence de Rh+Ir montrent une très bonne stabilité du système catalytique -absence de dépôt métallique-contrairement à ceux réalisés avec Rh seul.

Les conditions et résultats sont rassemblés dans le tableau 1 joint en annexe.

## 2.2 Concentration en métaux de corrosion 0 à 4000 mg/kg

Ces essais sont réalisés selon le mode opératoire-type donné en 1.1.

Pour chaque rapport molaire Rh/Ir entre 100/0 et 30/70 la concentration en métaux de corrosion est étudiée dans l'intervalle 0-4000 mg/kg.

L'ensemble des conditions et des résultats des différents essais réalisés sont rassemblés dans le tableau 2 joint en annexe.

Tous les essais réalisés avec [MétCor] = 0 sont des essais comparatifs.

2.2.1 Essais avec [Rh] = constante ; [Ir] = variable

➤ Rapport molaire Rh/Ir = 100/0 [Rh] = 0,33 à 0,34 mmole [Ir] = 0

Essais comparatifs, hors invention car [Ir] = 0 utilisés comme références

➤ Rapport molaire Rh/Ir = 90/10; [Rh] = 0,33 à 0,34 mmole ; [Ir] ≃ 0,04 mmole
➤ Rapport molaire Rh/Ir = 80/20; [Rh] = 0,33 à 0,34 mmole ; [Ir] ≃ 0,08-0,09 mmole
➤ Rapport molaire Rh/Ir = 70/30 ; [Rh] = 0,33 à 0,34 mmole ; [Ir] ≃ 0,14 mmole
➤ Rapport molaire Rh/Ir = 50/50 ; [Rh] = 0,33 à 0,34 mmole ; [Ir] ≃ 0,33 mmole
➤ Rapport molaire Rh/Ir = 30/70 ; [Rh] = 0,33 à 0,34 mmole; [Ir] ≃ 0,8 mmole

De tous ces essais, on peut conclure que :

⇒ Pour les rapports molaires Rh/Ir entre 90/10 et 50/50 (exclu), la vitesse de carbonylation du système Rh+Ir est conservée par rapport à celle du système Rh seul (Rh/Ir = 100/0) et cela quelles que soient les concentrations en métaux de corrosion entre 0 et 4000 mg/kg (entre 5 et 6 moles/l.h).

⇒ Pour les rapports molaires Rh/Ir de 50/50 à 30/70, la vitesse de carbonylation du système Rh+Ir diminue lorsque la concentration en métaux de corrosion augmente de 0 à 4000 mg/kg (pour Rh/Ir = 50/50 de 7,5 à 5,5 moles/l.h et pour Rh/Ir = 30/70 de 10 à 8 moles/l.h).

⇒ Stabilité du système catalytique :

Pour les essais au rhodium seul, présence d'un dépôt métallique sauf pour une concentration en métaux de corrosion de 4000 mg/kg.

Pour tous les autres essais en présence de Rh+Ir, absence de dépôt métallique, quelle que soit la concentration en métaux de corrosion.

2.2.2 Essais avec [Rh]+[Ir] = constantes = 0,33-0,34 mmole

➤ Rapport molaire Rh/Ir = 100/0 [Rh] = 0,33-0,34 mmole est à nouveau une série de référence d'essais comparatifs car [Ir] = 0
➤ Rapport molaire Rh/Ir = 70/30 ; [Rh] ≃ 0,23 mmole ; [Ir] ≃ 0,11 mmole
➤ Rapport molaire Rh/Ir = 50/50 ; [Rh] ≃ 0,165 mmole ; [Ir] ≃ 0,166 mmole
➤ Rapport molaire Rh/Ir = 30/70 ; [Rh] ≃ 0,10 mmole ; [Ir] ≃ 0,23 mmole

On constate que :

⇒ Pour les rapports molaires Rh/Ir entre 70/30 et 50/50 (inclus), la vitesse de carbonylation du système Rh+Ir n'est que peu ou pas altérée par l'augmentation de la concentration des métaux de corrosion de 0 à 4000 mg/kg (ou du moins pas plus que les références Rh/Ir = 100/0).

Cette légère perte de vitesse de carbonylation lorsque [MétCor] passe de 0 à 4000 mg/kg est estimée à 5 % pour Rh/Ir = 100/0, 12 % pour 70/30, 0 % pour 50/50 et 33 % dans les essais Rh/Ir = 30/70.

$\Rightarrow$ Stabilité du système catalytique :

Mêmes constatations qu'au paragraphe 2.2.1.

## 3. ESSAIS AVEC UN MILIEU REACTIONNEL INDUSTRIEL

Ces essais sont réalisés en appliquant le mode opératoire-type donné en 1.2.

### 3.1 Optimisation de la quantité de méthanol introduite

Dans cette série d'essais comparatifs (absence d'iridium) on a fait varier entre 5 et 15 % la quantité de méthanol ajouté (10 % du milieu réactionnel dans le mode opératoire type). Tous les autres paramètres sont identiques en particulier [Rh] = 0,33-0,34 mmole, [MétCor] = 5000 mg/kg, et [Eau] = 14%. La vitesse de carbonylation maximum - 7 moles/l.h est obtenue pour 10 % de méthanol ajouté ; elle diminue très légèrement quand la quantité de méthanol ajouté augmente à 12 voire 15 % (6 moles/l.h).

Les conditions et les résultats de ces essais sont consignés dans le tableau 3.1.

### 3.2 Essais avec [Rh] = constante ; [Ir] = variable

Dans cette série d'essais dont les résultats et les conditions sont rassemblés dans le tableau 3.2 ci-après, on a réalisé des ajouts croissants d'iridium à un milieu réactionnel industriel initial de composition constante, en particulier [Eau] = 14 %, [MétCor] = 5000 mg/kg et [Rh] = 0,33-0,34 millimole.

Les rapports molaires Rh/Ir sont ajustés par addition croissante d'iridium :

> Rh/Ir = 100/0 Essais comparatifs (absence d'iridium)
> Rh/Ir = 90/10, 80/20, 70/30, 50/50 et 30/70 Essais selon l'invention

Remarque 1 : Les essais pour un même rapport Rh/Ir sont en général doublés.

Remarque 2 : Dans l'essai 602 (Rh/Ir = 30/70) les 10 % de méthanol ajoutés sont remplacés par 25 % d'acétate de méthyle ; aucune influence sur Vcarb n'est observée.

$\Rightarrow$ On peut ajouter des quantités croissantes d'iridium à un milieu réactionnel industriel réel (conventionnel, catalyse rhodium seul) sans perte de production ni perte de stabilité du système catalytique.

- la vitesse de carbonylation pour Rh seul (6 à 7 moles/l.h) est conservée jusqu'aux rapports molaires Rh/Ir = 50/50 voire améliorée pour Rh/Ir = 30/70 (Vcarb = 8-8,5 moles/l.h).
- Aucun dépôt métallique n'est observé quels que soient les rapports molaires Rh/Ir entre 100/0 et 30/70.

### 3.3 Influence de la concentration en eau et du rapport Rh/Ir

Le mode opératoire type 1-2 est appliqué avec la variante suivante :

Pour les essais qui le nécessitent, la concentration en eau est ajustée à x % (x = 12 % ou 10 % ou 8 % ou 6 %) par introduction de la quantité d'anhydride acétique calculée par rapport à la concentration d'eau initiale de 14 % (14-x %) et la prise d'essai initiale de 50 ml de milieu réactionnel industriel ; cette quantité d'anhydride acétique est introduite dans les 50 ml de milieu réactionnel, au tout début de l'essai, avant fermeture de l'autoclave et pressurisation par 5 bar de monoxyde de carbone. Le mode opératoire type est alors poursuivi.

Dans ces 4 séries d'essais sont maintenues constantes la composition initiale du milieu réactionnel dont [MétCor] = 5000 mg/kg et pratiquement la concentration en catalyseur [Rh] + [Ir] = 0,33 à 0,44 millimole (A noter dans la 4$^{\text{ème}}$ série -essais 734 à 739- la quantité de milieu réactionnel est réduite à 43 ml/48 g et donc le méthanol ajouté est 4,8 g).

Les conditions et résultats des essais sont rassemblés dans le tableau 3.3 donné en annexe.

> Rapport molaire Rh/Ir = 100/0 ; [Rh] = 0,33-0,34 mmole ; [Ir] = 0

essais comparatifs hors invention car [Ir] = 0 utilisés comme références

➢ Rapport molaire Rh/Ir = 90/10 ; [Rh] = 0,33-0,34 mmole ; [Ir] ≃ 0,04 mmole

➢ Rapport molaire Rh/Ir = 80/20 ; [Rh] = 0,35-0,36 mmole ; [Ir] ≃ 0,09 mmole

➢ Rapport molaire Rh/Ir = 70/30 ; [Rh] = 0,23-0,24 mmole ; [Ir] ≃ 0,10 mmole

Donc [Rh] + [Ir] = 0,33-0,44 mmole.

Ces différents essais sont directement comparables car [Rh] + [Ir] sont pratiquement constantes, en particulier les séries Rh/Ir = 100/0 et 70/30 où [Rh] + [Ir] sont identiques à 0,33 - 0,34 mmole.

Globalement, on peut conclure que :

⇒ la diminution de la teneur en eau conduit à la diminution de la vitesse de carbonylation pour chaque série, quels que soient les rapports molaires Rh/Ir entre 100/0 et 70/30.

⇒ pratiquement pour chaque concentration en eau, les vitesses de carbonylation se classent dans l'ordre suivant Rh/Ir = 100/0 ≤ Rh/Ir = 90/10 ≤ Rh/Ir = 80/20 < Rh/Ir = 70/30

⇒ L'abaissement de la concentration en eau de 14 % à 12 % voire 10 % ne s'accompagne que d'une très faible diminution de la vitesse de carbonylation : Vcarb (Rh/Ir = 70/30, eau = 12 %) = Vcarb (Rh/Ir = 100/0, eau = 14 %).

⇒ Pas d'influence (négative) visible de la présence des 5000 mg/kg en métaux de corrosion sur les additions croissantes d'iridium aussi bien en termes de vitesse de carbonylation que de stabilité du système catalytique Rh+Ir (absence de dépôt métallique).

## 4. <u>Mise en solution simultanée de rhodium et d'iridium</u>

Ces exemples décrivent la mise en solution simultanée de rhodium et d'iridium- sous formes d'iodure de rhodium et d'iodure d'iridium - dans des conditions dérivées des conditions classiques du type de celles décrites dans le procédé MONSANTO pour l'iodure de rhodium seul. Les solutions ainsi obtenues sont des solutions catalytiques destinées à effectuer des ajouts simultanés de ces deux catalyseurs - Rh et Ir - dans le milieu réactionnel de carbonylation.

Dans un autoclave de 100 ml en Hastelloy® B2, on introduit l'iodure de rhodium, l'iodure d'iridium, l'eau et l'acide acétique selon les quantités indiquées dans le tableau 4.1 indiquant les conditions opératoires. L'autoclave est pressurisé avec du CO à température ambiante et porté à 110 °C, la pression est ajustée et maintenue à $5.10^5$ Pascal (5 bar) avec du CO et la réaction poursuivie à 110°C pendant 5 ou 8 heures, selon l'essai.

L'autoclave est refroidi, la solution catalytique est collectée et pesée afin de s'assurer de l'absence de fuites et valider ainsi l'essai.

La solution catalytique est analysée : aspect, présence ou absence de dépôt, concentrations en rhodium et iridium par spectrométrie d'absorption atomique.

L'autoclave est également inspecté pour s'assurer de la présence ou de l'absence de dépôt métallique. Ces différents résultats d'analyses sont donnés dans le tableau 4-2.

L'aspect limpide des solutions catalytiques, ainsi que la comparaison des concentrations théoriques et dosées par analyse sur la solution finale, prouvent les mises en solution totales du rhodium et de l'iridium.

Il est donc possible de préparer des solutions catalytiques:

- concentrées - 950 mg de Rh/kg de solution et 950 à 2600 mg d'Ir/kg de solution (essais 780, 787 et 785)
- ou très concentrées dans l'essai 788 - 1 % poids de Rh et 1,4 % poids d'Ir »

## TABLEAU N° 1

| ESSAIS AVEC DES SOLUTIONS SYNTHETIQUES | | | | | | | | |
|---|---|---|---|---|---|---|---|---|
| CONCENTRATION EN METAUX DE CORROSION NULLE | | | | | | | | |
| Essai n° | Type essai | Dépôt métallique | MétCor mg/kg | Rh mmole | Ir mmole | Rh % molaire | Ir % molaire | Vcarb 10' mol/l.h |
| 606 | Comparatif | Présence | 0 | 0,334 | 0 | 100 | 0 | 5,5 |
| 719 | Comparatif | Présence | 0 | 0,337 | 0 | 100 | 0 | 4,5 |
| 610 | Comparatif | Absence | 0 | 0,333 | 0,037 | 90 | 10 | 5,3 |
| 611 | Comparatif | Absence | 0 | 0,336 | 0,084 | 80 | 20 | 6 |
| 721 | Comparatif | Absence | 0 | 0,334 | 0,145 | 70 | 30 | 5 |
| 726 | Comparatif | Absence | 0 | 0,334 | 0,332 | 50 | 50 | 7,5 |
| 730 | Comparatif | Absence | 0 | 0,333 | 0,777 | 30 | 70 | 10,5 |
| 599 | Comparatif | Absence | 0 | 0,334 | 0,801 | 30 | 70 | 10 |

**TABLEAU N° 2**

### ESSAIS AVEC DES SOLUTIONS SYNTHETIQUES
### CONCENTRATION EN METAUX DE CORROSION 0 à 4000 mg/kg

| Essai n° | Type essai | Dépôt métallique | MetCor mg/kg | Rh mmole | Ir mmole | Rh % molaire | Ir % molaire | Vcarb 10' Ir/Rh = 0/100 |
|---|---|---|---|---|---|---|---|---|
| 606 | Comparatif | Présence | 0 | 0,334 | 0 | 100 | 0 | 5,5 |
| 719 | Comparatif | Présence | 0 | 0,337 | 0 | 100 | 0 | 4,5 |
| 715 | Comparatif | Présence | 100 | 0,335 | 0 | 100 | 0 | 6 |
| 716 | Comparatif | Présence | 1000 | 0,333 | 0 | 100 | 0 | 5 |
| 717 | Comparatif | Présence | 2000 | 0,333 | 0 | 100 | 0 | 5 |
| 720 | Comparatif | Absence | 4000 | 0,335 | 0 | 100 | 0 | 5 |

| Essai n° | Type essai | Dépôt métallique | MetCor mg/kg | Rh mmole | Ir mmole | Rh % molaire | Ir % molaire | Vcarb 10' Ir/Rh = 10/90 |
|---|---|---|---|---|---|---|---|---|
| 610 | Comparatif | Absence | 0 | 0,333 | 0,037 | 90 | 10 | 5,3 |
| 612 | Selon Invention | Absence | 100 | 0,336 | 0,037 | 90 | 10 | 5 |
| 613 | Selon Invention | Absence | 1000 | 0,334 | 0,037 | 90 | 10 | 6 |
| 614 | Selon Invention | Absence | 2000 | 0,333 | 0,037 | 90 | 10 | 5,3 |
| 615 | Selon Invention | Absence | 4000 | 0,335 | 0,042 | 90 | 10 | 5,3 |

| Essai n° | Type essai | Dépôt métallique | MetCor mg/kg | Rh mmole | Ir mmole | Rh % molaire | Ir % molaire | Vcarb 10' Ir/Rh = 20/80 |
|---|---|---|---|---|---|---|---|---|
| 611 | Comparatif | Absence | 0 | 0,336 | 0,084 | 80 | 20 | 6 |
| 616 | Selon Invention | Absence | 100 | 0,335 | 0,083 | 80 | 20 | 6 |
| 617 | Selon Invention | Absence | 1000 | 0,335 | 0,089 | 80 | 20 | 6 |
| 618 | Selon Invention | Absence | 2000 | 0,334 | 0,085 | 80 | 20 | 5,5 |
| 622 | Selon Invention | Absence | 4000 | 0,334 | 0,083 | 80 | 20 | 6 |

| Essai n° | Type essai | Dépôt métallique | MetCor mg/kg | Rh mmole | Ir mmole | Rh % molaire | Ir % molaire | Vcarb 10' Ir/Rh = 30/70 |
|---|---|---|---|---|---|---|---|---|
| 721 | Comparatif | Absence | 0 | 0,334 | 0,145 | 70 | 30 | 5 |
| 722 | Selon Invention | Absence | 100 | 0,335 | 0,143 | 70 | 30 | 6 |
| 723 | Selon Invention | Absence | 4000 | 0,333 | 0,144 | 70 | 30 | 5,5 |
| 724 | Comparatif | Absence | 0 | 0,235 | 0,108 | 70 | 30 | 4 |
| 725 | Selon Invention | Absence | 4000 | 0,233 | 0,107 | 70 | 30 | 3,5 |

| Essai n° | Type essai | Dépôt métallique | MetCor mg/kg | Rh mmole | Ir mmole | Rh % molaire | Ir % molaire | Vcarb 10' Ir/Rh = 50/50 |
|---|---|---|---|---|---|---|---|---|
| 726 | Comparatif | Absence | 0 | 0,334 | 0,332 | 50 | 50 | 7,5 |
| 727 | Selon Invention | Absence | 4000 | 0,333 | 0,334 | 50 | 50 | 5,5 |
| 728 | Comparatif | Absence | 0 | 0,165 | 0,166 | 50 | 50 | 3 |
| 729 | Selon Invention | Absence | 4000 | 0,165 | 0,166 | 50 | 50 | 3 |

| Essai n° | Type essai | Dépôt métallique | MetCor mg/kg | Rh mmole | Ir mmole | Rh % molaire | Ir % molaire | Vcarb 10' Ir/Rh = 70/30 |
|---|---|---|---|---|---|---|---|---|
| 599 | Comparatif | Absence | 0 | 0,334 | 0,801 | 30 | 70 | 10 |
| 730 | Comparatif | Absence | 0 | 0,333 | 0,777 | 30 | 70 | 10,5 |
| 731 | Selon Invention | Absence | 4000 | 0,333 | 0,777 | 30 | 70 | 8 |
| 732 | Comparatif | Absence | 0 | 0,099 | 0,233 | 30 | 70 | 3 |
| 733 | Selon Invention | Absence | 4000 | 0,103 | 0,233 | 30 | 70 | 2 |

**Les vitesses de carbonylation sont exprimées en mole par litre et par heure = mol/l.h**

**TABLEAU 3-1**

**ESSAIS AVEC UN MILIEU REACTIONNEL INDUSTRIEL**
**OPTIMISATION DE LA QUANTITE DE METHANOL INTRODUITE**

| Essai n° | Type essai | MeOH % | MetCor mg/kg | Rh mmole | Ir mmole | Rh % molaire | Ir % molaire | Vcarb 10' mol/l.h |
|---|---|---|---|---|---|---|---|---|
| 583 | Comparatif | 5 | 5000 | 0,336 | 0 | 100 | 0 | 2 |
| 581 | Comparatif | 10 | 5000 | 0,329 | 0 | 100 | 0 | 7 |
| 604 | Comparatif | 12 | 5000 | 0,329 | 0 | 100 | 0 | 6,4 |
| 605 | Comparatif | 15 | 5000 | 0,327 | 0 | 100 | 0 | 6 |

**· TABLEAU 3-2**

**ESSAIS AVEC UN MILIEU REACTIONNEL INDUSTRIEL**
**ESSAIS AVEC [Rh] = CONSTANTE ; [Ir] = VARIABLE**

| Essai n° | Type essai | Dépôt métallique | MetCor mg/kg | Rh mmole | Ir mmole | Rh % molaire | Ir % molaire | Vcarb 10' mol/l.h |
|---|---|---|---|---|---|---|---|---|
| 581 | Comparatif | Absence | 5000 | 0,329 | 0 | 100 | 0 | 7 |
| 603 | Comparatif | Absence | 5000 | 0,341 | 0 | 100 | 0 | 7 |
| 655 | Comparatif | Absence | 5000 | 0,329 | 0 | 100 | 0 | 6,5 |
| 656 | Selon Invention | Absence | 5000 | 0,338 | 0,037 | 90 | 10 | 7 |
| 661 | Selon Invention | Absence | 5000 | 0,332 | 0,038 | 90 | 10 | 6,5 |
| 657 | Selon Invention | Absence | 5000 | 0,338 | 0,085 | 80 | 20 | 6,5 |
| 662 | Selon Invention | Absence | 5000 | 0,333 | 0,083 | 80 | 20 | 6 |
| 664 | Selon Invention | Absence | 5000 | 0,331 | 0,143 | 70 | 30 | 6,5 |
| 663 | Selon Invention | Absence | 5000 | 0,339 | 0,144 | 70 | 30 | 7 |
| 659 | Selon Invention | Absence | 5000 | 0,338 | 0,336 | 50 | 50 | 7 |
| 660 | Selon Invention | Absence | 5000 | 0,335 | 0,782 | 30 | 70 | 8,5 |
| 602 (25 % AcOMe) | Selon Invention | Absence | 5000 | 0,333 | 0,774 | 30 | 70 | 8 |

**TABLEAU 3-3**

**ESSAIS AVEC UN MILIEU REACTIONNEL INDUSTRIEL**
**INFLUENCE DE LA CONCENTRATION EN EAU ET DU RAPPORT Rh / Ir**

| Essai n° | Type essai | Dépôt métallique | Eau % | Rh mmole | Ir mmole | Rh % molaire | Ir % molaire | Vcarb 10' mol/l.h |
|---|---|---|---|---|---|---|---|---|
| 700 | Comparatif | Absence | 14 | 0,338 | 0 | 100 | 0 | 7 |
| 699 | Comparatif | Absence | 12 | 0,339 | 0 | 100 | 0 | 6 |
| 698 | Comparatif | Absence | 10 | 0,332 | 0 | 100 | 0 | 4,5 |
| 696 | Comparatif | Absence | 8 | 0,335 | 0 | 100 | 0 | 4 |
| 697 | Comparatif | Absence | 6 | 0,342 | 0 | 100 | 0 | 3 |

| Essai n° | Type essai | Dépôt métallique | Eau % | Rh mmole | Ir mmole | Rh % molaire | Ir % molaire | Vcarb 10' mol/l.h |
|---|---|---|---|---|---|---|---|---|
| 701 | Selon Invention | Absence | 14 | 0,332 | 0,038 | 90 | 10 | 7 |
| 702 | Selon Invention | Absence | 12 | 0,343 | 0,038 | 90 | 10 | 6 |
| 706 | Selon Invention | Absence | 10 | 0,331 | 0,039 | 90 | 10 | 5 |
| 704 | Selon Invention | Absence | 8 | 0,343 | 0,038 | 90 | 10 | 4,3 |
| 705 | Selon Invention | Absence | 6 | 0,335 | 0,038 | 90 | 10 | 3,2 |

| Essai n° | Type essai | Dépôt métallique | Eau % | Rh mmole | Ir mmole | Rh % molaire | Ir % molaire | Vcarb 10' mol/l.h |
|---|---|---|---|---|---|---|---|---|
| 707 | Selon Invention | Absence | 14 | 0,355 | 0,088 | 80 | 20 | 6,5 |
| 708 | Selon Invention | Absence | 12 | 0,346 | 0,088 | 80 | 20 | 6 |
| 709 | Selon Invention | Absence | 10 | 0,352 | 0,086 | 80 | 20 | 5 |
| 710 | Selon Invention | Absence | 8 | 0,353 | 0,088 | 80 | 20 | 4 |
| 711 | Selon Invention | Absence | 6 | 0,360 | 0,086 | 80 | 20 | 4 |

| Essai n°. | Type essai | Dépôt métallique | Eau % | Rh mmole | Ir mmole | Rh % molaire | Ir % molaire | Vcarb 10' mol/l.h |
|---|---|---|---|---|---|---|---|---|
| 734 | Selon Invention | Absence | 14 | 0,233 | 0,100 | 70 | 30 | 7,5 |
| 738 | Selon Invention | Absence | 12 | 0,235 | 0,100 | 70 | 30 | 7 |
| 736 | Selon Invention | Absence | 10 | 0,233 | 0,102 | 70 | 30 | 5,6 |
| 737 | Selon Invention | Absence | 8 | 0,233 | 0,101 | 70 | 30 | 4,3 |
| 739 | Selon Invention | Absence | 6 | 0,233 | 0,101 | 70 | 30 | 4 |

**TABLE 4-1**

**MISES EN SOLUTION SIMULTANEE DE RHODIUM ET D' IRIDIUM**
**TABLEAU DES CONDITIONS OPERATOIRES**

| Essai n° | Eau g | Acide acétique g | Iodure d'iridium g | Iodure de rhodium g | Temps réaction heure | Pression CO bar | Température °C |
|---|---|---|---|---|---|---|---|
| 780 | 15,74 | 58,04 | 0,228 | 0,329 | 5 | 5 | 110 |
| 787 | 15,74 | 58,04 | 0,423 | 0,332 | 5 | 5 | 110 |
| 785 | 15,74 | 58,04 | 0,639 | 0,329 | 5 | 5 | 110 |
| 788 | 9,00 | 28,22 | 2,000 | 2,000 | 8 | 5 | 110 |

**TABLE 4-2**

| MISES EN SOLUTION SIMULTANEE DE RHODIUM ET D' IRIDIUM | | | | | |
|---|---|---|---|---|---|
| TABLEAU DES RESULTATS | | | | | |
| Essai n° | Iridium théorique mg/kg | Iridium dosé mg/kg | Rhodium théorique mg/kg | Rhodium dosé mg/kg | Aspect solution après réaction |
| 780 | 941 | 875 | 942 | 917 | Solution jaune-marron, limpide Absence de dépôt autoclave |
| 787 | 1742 | 1680 | 948 | 845 | Solution jaune-marron limpide Absence de dépôt autoclave |
| 785 | 2624 | 2560 | 937 | 871 | Solution jaune-marron, limpide Absence de dépôt autoclave |
| 788 | 14892 | 14040 | 10324 | 9421 | Solution jaune-marron, limpide Absence de dépôt autoclave |

## Revendications

1. Procédé de fabrication en continu d'acide acétique et/ou d'acétate de méthyle, **caractérisé en ce que**, à partir d'un procédé industriel continu, dit procédé initial, de carbonylation du méthanol ou d'un dérivé carbonylable du méthanol, tel que le diméthyléther, les halogénures de méthyle ou l'acétate de méthyle, en phase liquide homogène et sous pression de monoxyde de carbone, en présence d'un système catalytique comprenant un catalyseur homogène à base de rhodium et un promoteur halogéné, et en présence d'une concentration en eau dans le milieu réactionnel supérieure ou égale à 14 %, on réalise pendant le fonctionnement continu de l'installation, une modification progressive de la composition dudit catalyseur homogène par additions au cours du temps, d'un composé d'iridium.

2. Procédé selon la revendication 1, **caractérisé en ce que** l'on poursuit ensuite la fabrication en continu de l'acide acétique et/ou de l'acétate de méthyle en maintenant la composition du système catalytique telle que modifiée.

3. Procédé selon la revendication 2, **caractérisé en ce que** l'on maintient les concentrations en rhodium et en iridium dans le milieu réactionnel, par ajout de composés de rhodium ou de composés d'iridium ou des deux à la fois.

4. Procédé selon la revendication 3, **caractérisé en ce que** l'on maintient les concentrations en rhodium et en iridium dans le milieu réactionnel par ajout simultané de rhodium et d'iridium au moyen d'une solution catalytique contenant du rhodium et de l'iridium en solution.

5. Procédé selon l'une des revendications 1 à 3, **caractérisé en ce que** lesdites additions de composé d'iridium sont réalisées sous forme d'iridium préalablement solubilisé.

6. Procédé selon la revendication 5, **caractérisé en ce que** l'on utilise un mode opératoire de mise en solution du composé d'iridium ajouté compatible avec les installations et procédé mis en oeuvre dans les procédés conventionnels de carbonylation.

7. Procédé selon l'une des revendications 1 à 4, **caractérisé en ce que** lesdites additions de composé d'iridium sont réalisées sous forme d'un dérivé solide de l'iridium que l'on solubilise in situ dans le milieu réactionnel.

8. Procédé selon l'une des revendications 1 à 7, **caractérisé en ce que** lesdites additions de composé d'iridium sont réalisées, de façon à compenser, les pertes en rhodium en remplaçant le rhodium perdu par de l'iridium.

9. Procédé selon l'une des revendications 1 à 8, **caractérisé en ce que** lesdites additions de composé d'iridium sont réalisées en continu ou en discontinu.

10. Procédé selon l'une des revendications 1 à 9, **caractérisé en ce que** l'on maintient dans le milieu réactionnel une concentration molaire en rhodium comprise entre 0,1 et 50 mmole/l et une concentration molaire en iridium comprise entre 0,1 et 25 mmole/l.

11. Procédé selon l'une des revendications 1 à 10, **caractérisé en ce que** le rapport atomique iridium/rhodium est maintenu à une valeur comprise entre 1/99 et 99/1.

**12.** Procédé selon l'une des revendications 1 à 9, **caractérisé en ce que** lesdites additions de composé d'iridium sont réalisées jusqu'à obtention d'iridium comme seul métal dans le système catalytique homogène.

**13.** Procédé selon l'une des revendications 1 à 12, **caractérisé en ce qu'**il est mis en oeuvre dans une installation comprenant une zone I de réaction où la température est maintenue à une valeur comprise entre 150 et 250°C et la pression totale est comprise entre $5.10^5$ Pa et $200.10^5$Pa, une zone II de flash où le produit issu de la zone I de réaction est partiellement vaporisé à une pression inférieure à celle régnant dans la zone I de réaction, la fraction non vaporisée issue de cette zone II de flash étant recyclée vers la zone I de réaction et la fraction vaporisée étant purifiée dans une zone III de séparation par distillation et purification de l'acide acétique et/ou de l'acétate de méthyle formés.

**14.** Procédé selon la revendication 13, **caractérisé en ce que** les additions dudit composé d'iridium sont réalisées par injection dans le milieu réactionnel de la zone I de réaction.

**15.** Procédé selon la revendication 13, **caractérisé en ce que** lesdites additions de composé d'iridium sont réalisées par injection dans un flux recyclé des zones II ou III vers la zone I de réaction.

**16.** Procédé selon l'une des revendications 1 à 15, **caractérisé en ce que** l'on contrôle la concentration en métaux de corrosion dans le milieu réactionnel, de façon à la maintenir inférieure ou égale à 5000 mg/kg.

**17.** Procédé selon l'une des revendications 1 à 16, **caractérisé en ce que** l'on abaisse la teneur en eau du milieu réactionnel à une valeur inférieure à 14 % en poids par rapport au poids du milieu réactionnel.

**18.** Procédé selon la revendication 17, **caractérisé en ce que** l'on maintient la concentration en eau dans le milieu réactionnel à une valeur inférieure à 5 % en poids par rapport au poids du milieu réactionnel.

**19.** Procédé selon la revendication 18, **caractérisé en ce que** l'on introduit du formiate de méthyle dans le milieu réactionnel et **en ce que** l'on réalise simultanément les réactions de carbonylation du méthanol ou du dérivé carbonylable du méthanol et d'isomérisation du formiate de méthyle sous pression de monoxyde de carbone.

**20.** Procédé selon l'une des revendications 18 ou 19, **caractérisé en ce que** l'on arrête progressivement l'introduction du méthanol ou du dérivé carbonylable de méthanol dans le milieu réactionnel, de façon à passer à un système d'isomérisation seule du formiate de méthyle, sous pression de monoxyde de carbone.

**21.** Procédé selon l'une des revendications 1 à 20, **caractérisé en ce qu'**il comprend en outre, une étape de modification progressive de la composition du catalyseur homogène par addition, au cours du temps, d'un composé du platine, la concentration en platine étant maintenue à une valeur comprise entre 1 mmole et 25 mmole par litre de milieu réactionnel.

**Patentansprüche**

**1.** Verfahren zur kontinuierlichen Herstellung von Essigsäure und/oder Methylacetat,
   **dadurch gekennzeichnet,**
   **daß** man aus einem kontinuierlichen industriellen Verfahren, dem Startverfahren, zur Carbonylierung von Methanol oder einem carbonylierbarem Methanolderivat wie Dimethylether, Methylhalogeniden oder Methylacetat, in homogener Flüssigphase und unter Kohlenmonoxiddruck, in Gegenwart eines Katalysatorsystems, das einen Rhodium-basierten homogenen Katalysator und einen halogenierten Promoter umfaßt und in Gegenwart einer Wasserkonzentration größer oder gleich 14% im Reaktionsmedium, während des kontinuierlichen Betriebs der Anlage eine fortschreitende Modifikation der Zusammensetzung des homogenen Katalysators durch im Laufe der Zeit erfolgendes Zugeben einer Iridiumverbindung durchführt.

**2.** Verfahren nach Anspruch 1, **dadurch gekennzeichnet, daß** anschließend die kontinuierliche Herstellung von Essigsäure und/oder Methylacetat fortgesetzt wird, während die Zusammensetzung des Katalysatorsystems, so wie sie modifiziert ist, beibehalten wird.

3. Verfahren nach Anspruch 2, **dadurch gekennzeichnet, daß** die Konzentrationen von Rhodium und Iridium in dem Reaktionsmedium beibehalten werden durch Zugeben von Rhodiumverbindungen oder Iridiumverbindungen oder von beiden gleichzeitig.

4. Verfahren nach Anspruch 3, **dadurch gekennzeichnet, daß** die Konzentrationen von Rhodium und Iridium in dem Reaktionsmedium beibehalten werden durch die gleichzeitige Zugabe von Rhodium und Iridium mittels einer katalytischen Lösung, die Rhodium und Iridium in Lösung enthält.

5. Verfahren nach einem der Ansprüche 1 bis 3, **dadurch gekennzeichnet, daß** die Zugaben an Iridiumverbindung in Form von vorsolubilisiertem Iridium durchgeführt werden.

6. Verfahren nach Anspruch 5, **dadurch gekennzeichnet, daß** eine Vorgehensart zum Lösen der zugegebenen Iridiumverbindung angewandt wird, die kompatibel mit den Anlagen und Verfahren ist, die in den konventionellen Carbonylierungsverfahren eingesetzt werden.

7. Verfahren nach einem der Ansprüche 1 bis 4, **dadurch gekennzeichnet, daß** die Zugaben an Iridiumverbindung in Form von einem festen Iridiumderivat durchgeführt werden, das in situ im Reaktionsmedium gelöst wird.

8. Verfahren nach einem der Ansprüche 1 bis 7, **dadurch gekennzeichnet, daß** die Zugaben einer Iridiumverbindung so durchgeführt werden ,daß die Verluste an Rhodium ausgeglichen werden, wobei das verlorene Rhodium mit Iridium ersetzt wird.

9. Verfahren nach einem der Ansprüche 1 bis 8, **dadurch gekennzeichnet, daß** die Zugaben an Iridiumverbindung kontinuierlich oder diskontinuierlich durchgeführt werden.

10. Verfahren nach einem der Ansprüche 1 bis 9, **dadurch gekennzeichnet, daß** im Reaktionsmedium eine molare Rhodiumkonzentration zwischen 0,1 und 50 mmol/l und eine molare Iridiumkonzentration zwischen 0,1 und 25 mmol/l beibehalten wird.

11. Verfahren nach einem der Ansprüche 1 bis 10, **dadurch gekennzeichnet, daß** das Iridium/Rhodium-Atomverhältnis auf einem Wert zwischen 1/99 und 99/1 gehalten wird.

12. Verfahren nach einem der Ansprüche 1 bis 9, **dadurch gekennzeichnet, daß** die Zugaben einer Iridiumverbindung bis zum Erhalt von Iridium als einziges in dem homogenen Katalysatorsystem vorliegendes Metall erfolgen.

13. Verfahren nach einem der Ansprüche 1 bis 12, **dadurch gekennzeichnet, daß** es in einer Anlage ausgeführt wird, die eine Reaktionszone I, wo die Temperatur bei einem Wert zwischen 150 und 250°C gehalten wird und der Gesamtdruck zwischen $5 \cdot 10^5$ Pa und $200 \cdot 10^5$ Pa liegt, und eine Flashzone II umfaßt, wo das Produkt aus der Reaktionszone I teilweise bei einem Druck unter jenem verdampft wird, der in der Reaktionszone I herrscht, wobei die nicht verdampfte Fraktion aus der Flashzone II zur Reaktionszone I und die verdampfte Fraktion in einer Zone III zur Trennung durch Destillation und Reinigung der Essigsäure und/oder des Methylacetats, die gebildet werden, rezykliert wird.

14. Verfahren nach Anspruch 13, **dadurch gekennzeichnet, daß** die Zugaben der Iridiumverbindung durch Einspritzen in das Reaktionsmedium der Reaktionszone I durchgeführt werden.

15. Verfahren nach Anspruch 13, **dadurch gekennzeichnet, daß** die Zugaben an Iridiumverbindung durch Einspritzen in einen Strom durchgeführt werden, der von den Zonen II oder III in die Reaktionszone I rezykliert ist.

16. Verfahren nach einem der Ansprüche 1 bis 15, **dadurch gekennzeichnet, daß** die Konzentration an Korrosionsmetallen im Reaktionsmedium überwacht wird, derart, daß sie unter oder gleich 5000 mg/kg gehalten wird.

17. Verfahren nach einem der Ansprüche 1 bis 16, **dadurch gekennzeichnet, daß** der Wassergehalt des Reaktionsmediums auf einen Wert unter 14 Gew.-% im Verhältnis zum Gewicht des Reaktionsmediums vermindert wird.

18. Verfahren nach Anspruch 17, **dadurch gekennzeichnet, daß** die Wasserkonzentration im Reaktionsmedium auf einem Wert unter 5 Gew.-% im Verhältnis zum Gewicht des Reaktionsmediums gehalten wird.

**19.** Verfahren nach Anspruch 18, **dadurch gekennzeichnet, daß** Methylformiat in das Reaktionsmedium eingeführt wird, und **dadurch**, daß die Reaktionen zur Carbonylierung von Methanol oder carbonylierbarem Methanolderivat und zur Isomerisierung von Methylformat gleichzeitig unter Kohlenmonoxiddruck durchgeführt werden.

**20.** Verfahren nach einem der Ansprüche 18 oder 19, **dadurch gekennzeichnet, daß** die Einführung von Methanol oder carbonylierbarem Methanolderivat in das Reaktionsmedium schrittweise derart angehalten wird, daß zu einem alleinigen System zur Isomerisierung des Methylformiats unter Kohlenmonoxiddruck übergegangen wird.

**21.** Verfahren nach einem der Ansprüche 1 bis 20, **dadurch gekennzeichnet, daß** es außerdem einen Schritt zum fortschreitenden Modifizieren der Zusammensetzung des homogenen Katalysators durch Zugabe einer Platinverbindung über die Zeit umfaßt, wobei die Platinkonzentration bei einem Wert zwischen 1 mmol und 25 mmol pro Liter Reaktionsmedium gehalten wird.

## Claims

**1.** Process for the continuous manufacture of acetic acid and/or methyl acetate, **characterized in that**, starting from a continuous industrial process, called the initial process, for the carbonylation of methanol or a carbonylatable derivative of methanol such as dimethyl ether, methyl halides or methyl acetate, in the homogeneous liquid phase and under carbon monoxide pressure, in the presence of a catalyst system comprising a rhodium-based homogeneous catalyst and a halogenated promoter, and in the presence of a concentration of water greater than or equal to 14% in the reaction medium, the composition of said homogeneous catalyst is modified gradually during the continuous operation of the installation by adding an iridium compound over time.

**2.** Process according to claim 1, **characterized in that** the continuous manufacture of acetic acid and/or methyl acetate is then continued while maintaining the composition of the catalyst system as modified.

**3.** Process according to claim 2, **characterized in that** the concentrations of rhodium and iridium in the reaction medium are maintained by adding rhodium compounds or iridium compounds or both at once.

**4.** Process according to claim 3, **characterized in that** the concentrations of rhodium and iridium in the reaction medium are maintained by the simultaneous addition of rhodium and iridium by means of a catalyst solution containing rhodium and iridium in solution.

**5.** Process according to one of claims 1 to 3, **characterized in that** said additions of iridium compound are effected in the form of presolubilized iridium.

**6.** Process according to claim 5, **characterized in that** the procedure used to solubilize the added iridium compound is compatible with the installations and process employed in conventional carbonylation processes.

**7.** Process according to one of claims 1 to 4, **characterized in that** said additions of iridium compound are effected in the form of a solid iridium derivative which is solubilized in situ in the reaction medium.

**8.** Process according to one of claims 1 to 7, **characterized in that** said additions of iridium compound are effected so as to compensate the losses of rhodium, the lost rhodium being replaced with iridium.

**9.** Process according to one of claims 1 to 8, **characterized in that** said additions of iridium compound are effected continuously or batchwise.

**10.** Process according to one of claims 1 to 9, **characterized in that** the molar concentrations of rhodium and iridium which are maintained in the reaction medium are between 0.1 and 50 mmol/l and between 0.1 and 25 mmol/l respectively.

**11.** Process according to one of claims 1 to 10, **characterized in that** the iridium/rhodium atomic ratio is kept at a value of between 1/99 and 99/1.

**12.** Process according to one of claims 1 to 9, **characterized in that** said additions of iridium compound are effected until iridium is the only metal present in the homogeneous catalyst system.

13. Process according to one of claims 1 to 12, **characterized in that** it is carried out in an installation comprising a reaction zone I, where the temperature is maintained at a value of between 150 and 250°C and the total pressure is between $5.10^5$ Pa and $200.10^5$ Pa, and a flash zone II, where the product coming from the reaction zone I is partially vaporized at a pressure below that prevailing in the reaction zone I, the non-vaporized fraction coming from this flash zone II being recycled into the reaction zone I and the vaporized fraction being purified in a zone III, by separation/distillation/purification of the acetic acid and/or methyl acetate formed.

14. Process according to claim 13, **characterized in that** the additions of said iridium compound are effected by injection into the reaction medium of the reaction zone I.

15. Process according to claim 13, **characterized in that** said additions of iridium compound are effected by injection into a stream recycled from the zone II or III into the reaction zone I.

16. Process according to one of claims 1 to 15, **characterized in that** the concentration of corrosion metals in the reaction medium is monitored in order to be kept below or equal to 5000 mg/kg.

17. Process according to one of claims 1 to 16, **characterized in that** the water content of the reaction medium is reduced to a value below 14% by weight, based on the weight of reaction medium.

18. Process according to claim 17, **characterized in that** the concentration of water in the reaction medium is kept at a value below 5% by weight, based on the weight of reaction medium.

19. Process according to claim 18, **characterized in that** methyl formate is introduced into the reaction medium, and **in that** the carbonylation reaction of methanol or carbonylatable derivative of methanol and the methyl formate isomerization reaction are carried out simultaneously under carbon monoxide pressure.

20. Process according to one of claims 18 or 19, **characterized in that** the introduction of methanol or carbonylatable derivative of methanol into the reaction medium is stopped gradually in order to change to a system operating only with methyl formate isomerization under carbon monoxide pressure.

21. Process according to one of claims 1 to 20, **characterized in that** it also comprises a step for gradual modification of the composition of the homogeneous catalyst by the addition of a platinum compound over time, the concentration of platinum being kept at a value of between 1 mmole and 25 mmole per liter of reaction medium.